(19) Europäisches Patentamt
European Patent Office
Office européen des brevets

(11) Veröffentlichungsnummer: 0 253 258
A2

(12) **EUROPÄISCHE PATENTANMELDUNG**

(21) Anmeldenummer: 87109695.4

(22) Anmeldetag: 06.07.87

(51) Int. Cl.4: C07C 83/04 , C07C 83/08 , C07C 93/14 , C07C 93/26 , A23K 1/16

(30) Priorität: 16.07.86 DE 3623940

(43) Veröffentlichungstag der Anmeldung: 20.01.88 Patentblatt 88/03

(84) Benannte Vertragsstaaten: BE CH DE ES FR GB IT LI NL

(71) Anmelder: BAYER AG
Konzernverwaltung RP Patentabteilung
D-5090 Leverkusen 1 Bayerwerk(DE)

(72) Erfinder: Lindel, Hans, Dr.
Carl-Duisberg-Strasse 321
D-5090 Leverkusen 1(DE)
Erfinder: Ingendoh, Axel, Dr.
Engstenberger Höhe 10
D-5068 Odenthal(DE)
Erfinder: Berschauer, Friedrich, Dr.
Claudiusweg 9
D-5600 Wuppertal 1(DE)
Erfinder: De Jong, Anno, Dr.
Stockmannsmühle 46
D-5600 Wüppertal 1(DE)
Erfinder: Scheer, Martin, Dr.
Herberts-Katernberg 7
D-5600 Wuppertal 1(DE)

(54) **Arylethanolhydroxylamine, Verfahren zu ihrer Herstellung und ihre Verwendung zur Leistungsförderung.**

(57) Die vorliegende Erfindung betrifft Arylethanolhydroxylamine der Formel I

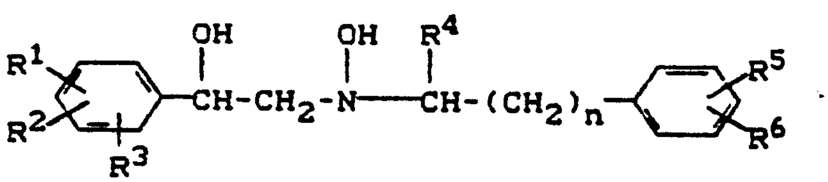

(I)

in welcher

$R^1$ für Halogen, CN, $NO_2$, Hydroxy, gegebenenfalls substituiertes Alkyl, Alkoxy oder Alkylthio ferner für Alkoxycarbonyl oder den Rest $-NR^7R^8$ steht,

$R^2$ für Wasserstoff, Halogen, CN, Hydroxy oder gegebenenfalls substituiertes Alkoxy steht,

$R^3$ für Wasserstoff, Halogen, Hydroxy oder gegebenenfalls substituiertes Alkoxy steht, wobei die Reste $R^2$

EP 0 253 258 A2

und $R^3$ gemeinsam mit den angrenzenden C-Atomen einen gegebenenfalls substituierten Dioxolanyl-oder Dioxanylring bilden können.

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy das gegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht.

Verfahren und Zwischenprodukte zu ihrer Herstellung sowie ihre Verwendung also Leistungsförderer für Tiere.

# Arylethanolhydroxylamine, Verfahren zu ihrer Herstellung und ihre Verwendung zur Leistungsförderung

Die vorliegende Erfindung betrifft Arylethanolhydroxylamine, Verfahren zu ihrer Herstellung und ihre Verwendung als Leistungsförderer für Tiere.

Arylethanolamine sind bekannte Verbindungen. Sie besitzen in Abhängigkeit von ihrer chemischen Struktur unterschiedliche pharmakologische Eigenschaften. Unter anderem besitzen bestimmte Arylethanolamine Wirkungen auf die Gewichtszunahme von Tieren sowie auf das Verhältnis von Fleisch zu Fett (EP-OS 26 298). Auch dabei scheint die Grundstruktur des Arylethanolamins von entscheidender Bedeutung für die Wirkung zu sein.

1. Es wurden die neuen Arylethanolhydroxylamine der Formel I

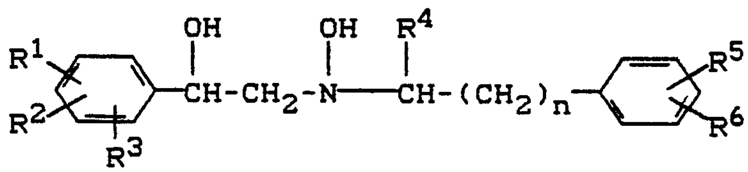

(I)

in welcher

$R^1$ für Halogen, CN, $NO_2$, Hydroxy, gegebenenfalls substituiertes Alkyl, Alkoxy oder Alkylthio, ferner für Alkoxycarbonyl oder den Rest $-NR^7R^8$ steht,

$R^2$ für Wasserstoff, Halogen, CN, Hydroxy oder gegebenenfalls substituiertes Alkoxy steht, wobei die Rests $R^1$ und $R^2$ gemeinsam mit den angrenzenden C-Atomen einen gegebenenfalls substituierten Dioxolanyl- oder Dioxanylring bilden können,

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht,

sowie deren physiologisch verträgliche Salze, Enantiomeren und Diastereomeren gefunden.

2. Es wurde ein Verfahren zur Herstellung der Arylethanolhydroxylamine der Formel I

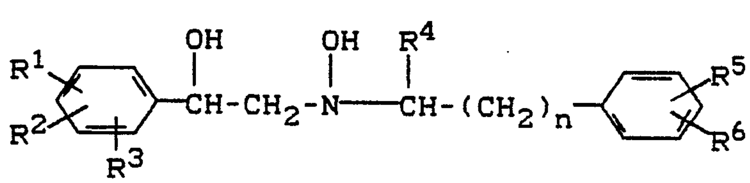

(I)

in welcher

$R^1$ für Halogen, CN, $NO_2$, Hydroxy, gegebenenfalls substituiertes Alkyl, Alkoxy oder Alkylthio, ferner für

Alkoxycarbonyl oder den Rest $-NR^7R^8$ steht,

$R^2$ für Wasserstoff, Halogen, CN, Hydroxy oder gegebenenfalls substituiertes Alkoxy steht, wobei die Rest $R^1$ und $R^2$ gemeinsam mit den angrenzenden C-Atomen einen gegebenenfalls substituierten Dioxolanyl- oder Dioxanylring bilden können,

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht,

gefunden, das dadurch gekennzeichnet ist, daß man Arylglyoxalnitrone der Formel II

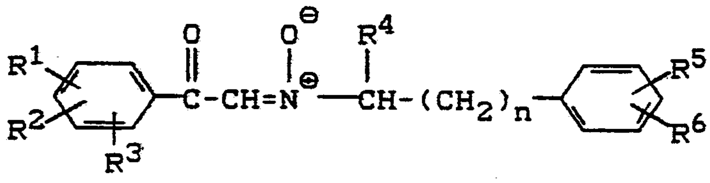

(II)

in welcher

$R^1$, $R^2$, $R^3$, $R^4$, $R^5$, $R^6$ und n die oben angegebene Bedeutung haben,

reduziert.

3. Es wurden die neuen Arylglyoxalnitrone der Formel II gefunden

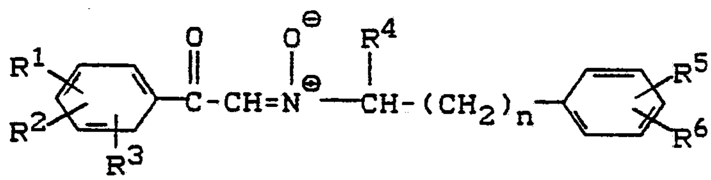

(II)

in welcher

$R^1$ für Halogen, CN, $NO_2$, Hydroxy, gegebenenfalls substituiertes Alkyl, Alkoxy oder Alkylthio, ferner für Alkoxycarbonyl oder den Rest $-NR^7R^8$ steht,

$R^2$ für Wasserstoff, Halogen, CN, Hydroxy oder gegebenenfalls substituiertes Alkoxy steht, wobei die Reste $R^1$ und $R^2$ gemeinsam mit den angrenzenden C-Atomen einen gegebenenfalls substituierten Dioxolanyl- oder Dioxanylring bilden können,

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht,

4. Es wurde ein Verfahren zur Herstellung der neuen Arylglyoxalnitrone der Formel II

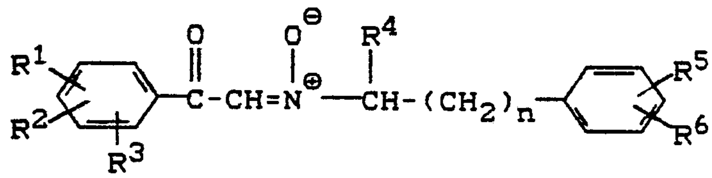

(II)

in welcher

$R^1$, $R^2$, $R^3$, $R^4$, $R^5$, $R^6$ und n die bei 3 (oben) angegebene Bedeutung haben,

gefunden, das dadurch gekennzeichnet ist, daß man Arylglyoxale der Formel III

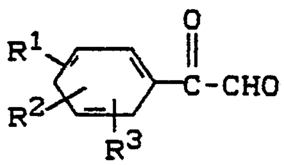

(III)

in welcher

$R^1$, $R^2$, $R^3$ die oben angegebene Bedeutung haben,

gegebenenfalls in Form ihrer Hydrate oder Addukte mit Alkoholen mit Hydroxylaminen der Formel IV

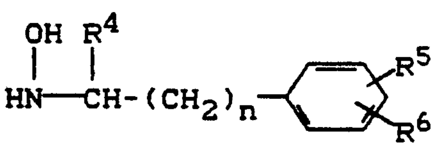

(IV)

in welcher

$R^4$, $R^5$, $R^6$, n die oben angegebene Bedeutung haben,

unter Wasserabspaltung umsetzt.

5. Es wurden die neuen Hydroxylamine der Formel IV gefunden

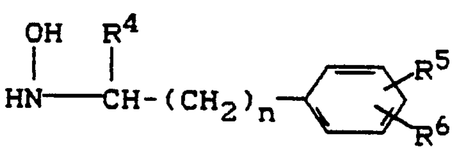

(IV)

in welcher

$R^4$, $R^5$, $R^6$, n die bei 3 (oben) angegebene Bedeutung haben.

6. Es wurde ein Verfahren zur Herstellung der neuen Hydroxylamine der Formel IV gefunden

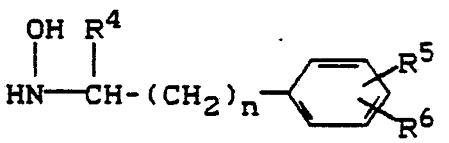

(IV)

in welcher

$R^4$, $R^5$, $R^6$, n die oben angegebene Bedeutung haben,

das dadurch gekennzeichnet ist, daß man Oxime der Formel V

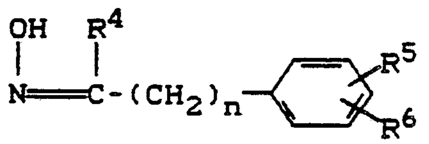

(V)

in welcher

$R^4$, $R^5$, $R^6$, n die oben angegebene Bedeutung haben,

reduziert.

Die Arylethanolhydroxylamine zeigen hervorragende leitungssteigernde Wirkung bei Tieren. Sie lassen sich daher auf dem Gebiet der Tierernährung als Leistungsförderer einsetzen. Diese Eigenschaft war erstaunlich. Es war nämlich bei Arylethanolaminen bekannt, daß Änderungen an der Substitution des Aminteils des Moleküls zu starken Änderungen der biologischen und pharmakologischen Wirkung führen. Es konnte daher keinesfalls erwartet werden, daß eine völlig andere chemische Klasse von Verbindungen, nämlich Hydroxylamine, wirtschaftlich verwertbare Wirkungen aufweisen.

Bevorzugt sind Verbindungen der Formel I, in welcher

$R^1$ für Fluor, Chlor, Brom, CN, Nitro, Hydroxy, $C_{1-4}$-Alkyl, $C_{1-4}$-Halogenalkyl, $C_{1-4}$-Hydroxyalkyl, $C_{1-4}$-Alkoxyalkyl, $C_{1-4}$-Alkoxy, $C_{1-4}$-Halogenalkoxy, Benzyloxy, $C_{1-4}$-Alkylthio, $C_{1-4}$-Halogenalkylthio, $C_{1-4}$-Alkoxycarbonyl, $C_{1-4}$-Alkylsulfonylmethyl oder den Rest $-NR^7R^8$ steht,

$R^2$ für Wasserstoff, Fluor, Chlor, Brom, CN, Hydroxy, $C_{1-4}$-Alkoxy steht, wobei die Reste $R^1$ und $R^2$ gemeinsam mit den C-Atomen, an die sie gebunden sind, einen gegebenenfalls durch Fluor oder Chlor substituierten Dioxolanyl-oder Dioxanylring bilden können,

$R^3$ für Wasserstoff, Fluor, Chlor oder Brom steht,

$R^4$ für Wasserstoff oder $C_{1-3}$-alkyl steht,

$R^5$ für Wasserstoff, Hydroxy, Fluor, Chlor, Brom, $C_{1-10}$-Alkyl, das gegebenenfalls durch Halogen, OH, CN, Phenyl, substituiert ist, oder für $C_{1-4}$-Alkoxy steht,

$R^6$ für $C_{1-3}$-Alkoxy, das gegebenenfalls durch OH, Halogen, $C_{1-2}$-Alkoxy, $-COR^9$ oder $-CONR^7R^8$ substituiert ist, oder für einen der folgenden Reste steht, $-COR^9$, $-SO_2R^{10}$, $-CONR^7R^8$ oder $-O-COR^9$,

$R^7$ für Wasserstoff, $C_{1-4}$-Alkyl, gegebenenfalls substituiertes Benzyl oder Phenyl, steht,

$R^8$ für Wasserstoff, $C_{1-4}$-Alkyl, -CHO, -$COR^9$, -$CONH_2$, -CONH ($C_{1-4}$-Alkyl), -CON($C_{1-4}$-Alkyl)$_2$, -$SO_2$-($C_{1-4}$-Alkyl) steht,

$R^9$ für Hydroxyl, $C_{1-4}$-Alkoxy, $C_{1-4}$-Alkoxy, $C_{1-4}$-Alkyl, gegebenenfalls substituiertes Phenyl steht.

$R^{10}$ für $C_{1-4}$-Alkyl oder $C_{1-4}$ -Halogenalkyl steht.

Besonders bevorzugt sind Verbindungen der Formel I, in welcher

$R^1$ für Chlor, Brom, CN, OH, Nitro, Methyl, Hydroxymethyl, Methyl, Methoxy, Methylsulfonylmethyl, Trifluormethyl, Amino, Formamido, Acetamido, Methylsulfonamido, Ureido, Methoxybenzylamino steht,

$R^2$ für Wasserstoff, Chlor, Brom, CN, Hydroxy, Methoxy steht,

$R^3$ für Wasserstoff, Chlor, Brom, steht,

$R^4$ für Wasserstoff oder Methyl steht,

$R^5$ für Wasserstoff, Methoxy, Ethoxy, die gegebenenfalls durch OH, substituiert sind, steht,

$R^6$ für Methoxy oder Ethoxy, die gegebenenfalls durch OH substituiert sind, oder für -$COR^9$, -O-$CH_2$-$COR^9$ steht,

$R^9$ für $C_{1-4}$ -Alkyl, $C_{1-4}$-Alkoxy insbesondere Methoxy, Ethoxy, Isopropoxy steht,

n für 1 steht.

Insbesondere seien Verbindungen der Formel I genannt, in welcher

$R^1$ für Chlor, Brom, CN, OH, Methylsulfonylmethyl, Hydroxy, Methoxy, Amino steht,

$R^2$ für Wasserstoff, Chlor, CN, Hydroxy, Methoxy steht,

$R^3$ für Wasserstoff, Chlor steht,

$R^4$ für Methyl steht,

$R^5$ für Wasserstoff steht,

$R^6$ für Methoxy, Hydroxyethyl oder -$COR^9$, -O-$CH_2COR^9$ steht,

$R^9$ für Methoxy, Isopropoxy steht,

n für 1 steht.

Im einzelnen seien neben den in den Beispielen genannten Verbindungen folgende Verbindungen der Formel I genannt:

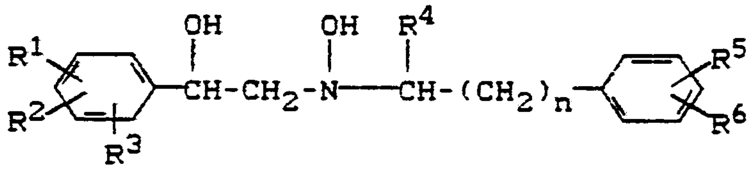

(I)

| $R^1$ | $R^2$ | $R^3$ | $R^4$ | n | $R^5$ | $R^6$ |
|---|---|---|---|---|---|---|
| 2-Br | H | H | $CH_3$ | 1 | H | 4-$COOCH_3$ |
| 3-Cl | 4-Cl | H | H | 2 | 3-Cl | 4-COOH |
| 3-$CH_3O$ | 4-$CH_3O$ | H | $CH_3$ | 2 | H | 4-$OCH_2COOCH_3$ |
| 4-$NH_2$ | 3-CN | H | $CH_3$ | 1 | H | 4-$OCH_2COOCH_3$ |

Die Verbindungen der Formel I können auch in Form ihrer sterischen und optischen Isomeren vorliegen und dabei zueinander enantiomere und/oder diastereomere Formen bilden.

Physiologisch verträgliche Salze der Verbindungen der Formel I können mit folgenden Säuren gebildet werden:

Salzsäure Schwefelsäure, Phosphorsäure, Perchlorsäure, Brom-, Iod-, Fluorwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäure, Ameisensäure, Chloressigsäure, Toluolsulfosäure, Benzolsulfonsäure, Trichloressigsäure, Phthalsäure, Naphthalinsulfonsäure, Nikotinsäure.

Die Verbindungen der Formel I werden erhalten, indem man Arylglyoxalnitrone der Formel II reduziert. Die Reaktion läßt sich durch folgendes Reaktionsschema darstellen:

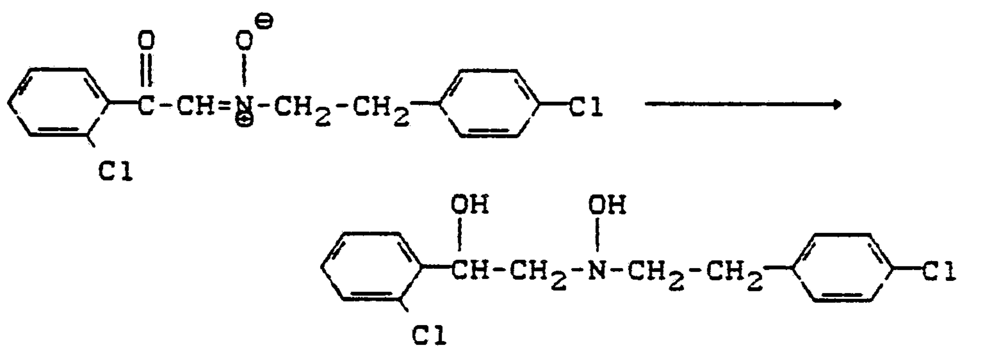

Es werden bevorzugt die Arylglyoxalnitrone der Formel II eingesetzt, in der die Reste $R^1$ bis $R^6$ und n die bei den Verbindungen der Formel I angegebene bevorzugten Bedeutungen haben.

Die Reduktion erfolgt bevorzugt mit komplexen Hydriden, z.B. Alkaliborhydriden wie z.B. $NaBH_4$, $NaBH_3CN$, $LiBH_4$, $NaBH_x$ $(O\text{-Alkyl})_y$, wobei x und y sich zu 4 ergänzen. Besonders bevorzugt ist dabei $NaBH_4$.

Die Umsetzung erfolgt bevorzugt in Verdünnungsmitteln. Hierzu zählen alle inerten organischen Lösungsmittel.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diglykoldimethylether, Tetrahydrofuran und Dioxan, weiterhin Alkohole, wie Methanol, Ethanol, Isopropanol oder höherkettige Alkohole, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Reaktion erfolgt bei Temperaturen von -50-100°C, vorzugsweise bei Temperaturen zwischen 0°C und 50°C.

Die Aufarbeitung erfolgt in üblicher Weise.

Arylglyoxalnitrone der Formel II sind neu. Sie werden erhalten, indem man Arylglyoxale der Formel III mit Hydroxylaminen der Formel IV umsetzt. Die Reaktion läßt sich durch folgendes Schema wiedergeben:

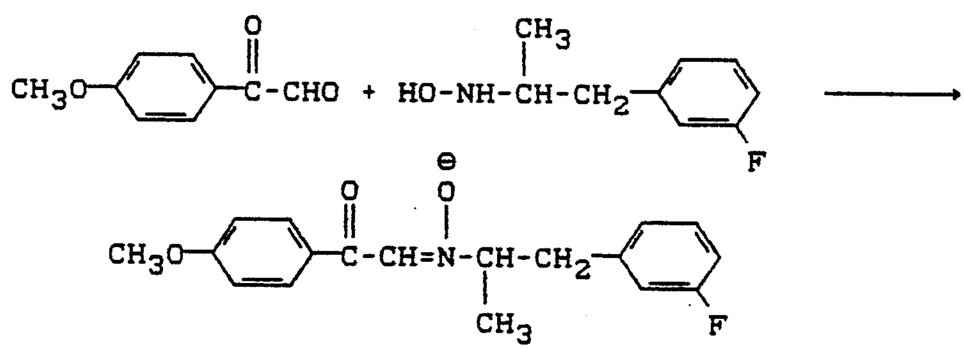

Arylflyoxale der Formel III sind bekannt oder lassen sich nach an sich bekannten Verfahren herstellen (N. Kornblum et.al., J. Am. Chem. Soc. 79 (1957), 6562), indem man z.B. die entsprechenden α-Halogenacetophenone z. B. mit Dimethylsulfoxid oxidiert, oder die entsprechenden Acetophenone mit Selendioxid oxidiert.

Es werden bevorzugt Aryglyoxale der Formel III eingesetzt, in der die Reste $R^1$, $R^2$, $R^3$ die bei den Verbindungen der Formel I angegebenen bevorzugten Bedeutungen haben.

Im einzelnen seien folgende Arylglyoxale genannt: 2-Chlorphenylglyoxal, 3-Chlorphenylglyoxal, 3-Bromphenylglyoxal, 3-Methylsulfonylmethyl-4-hydroxyphenylglyoxal, 3,4-Dimethoxyphenylglyoxal, 3,5-Dimethoxyphenylglyoxal.

Hydroxylamine der Formel IV sind neu. Ihre Herstellung wird weiter unten beschrieben.

Die Umsetzung kann mit oder ohne Verdünnungsmittel erfolgen. Als Verdünnungsmittel seien genannt:

Alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlor benzol, Alkohole wie Methanol, Ethanol, Isopropanol, Glycol und höherkettige Alkohole, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid und N-Methylpyrrolidon, sowie Dimethylsulfoxid, Tetramethylensulfon und Hexamethylphosphorsäuretriamid.

Vorzugsweise setzt man das N-Alkylhydroxylamin der allgemeinen Formel IV als Salz einer anorganischen oder organischen Säure mit dem Arylglyoxal der allgemeinen Formel III in Gegenwart eines Alkalisalzes einer schwachen organischen Säure wie z.B. Natriumacetat um.

Die Reaktion kann bei Temperaturen von 10-200°C, bevorzugt von 50-150°C, ganz besonders bevorzugt bei der Siedetemperatur des Lösungsmittels, erfolgen.

Es wird bevorzugt in Gegenwart eines wasserentziehenden Mittels gearbeitet. Als solche seien genannt z.B. $Na_2SO_4$, $MgSO_4$, $K_2CO_3$, $CaCl_2$, Molekularsiebe, Kieselgel, Aluminiumhydroxide. Das bei Reaktion entstehende Wasser kann auch azeotrop aus dem Reaktionsgemisch entfernt werden.

Die Verbindungen der Formeln III und IV werden bevorzugt im äquimolaren Verhältnis eingesetzt.

Die Aufbeitung erfolgt in an sich bekannter Weise.

Es muß der Herstellung von Arylethanolhydroxylaminen der allgemeinen Formel I nicht in jedem Fall das Arylglyoxalnitron der allgemeinen Formel II isoliert werden. Es kann sich als besonders günstig erweisen, die Reaktionsfolge in einer Eintopfreaktion durchzuführen.

Hydroxylamine der Formel IV sind neu.

Sie lassen sich herstellen, indem man Oxime der Formel V z.B. mit $NaBH_3CN$ reduziert. Die Reaktion läßt sich durch folgendes Formelschema darstellen:

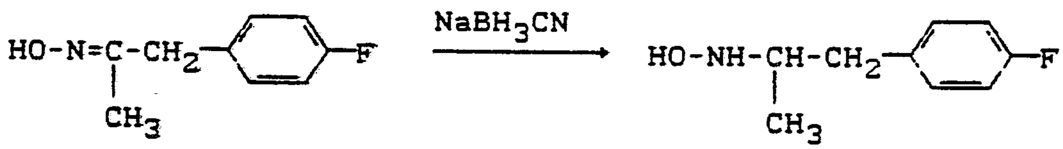


Oxime der Formel V sind bekannt (vgl. z.B. EP-OS 23 385) oder lassen sich analog zu bekannten Verfahren herstellen. Es werden bevorzugt die Oxime der Formel V eingesetzt, in der die Reste $R^4$ bis $R^6$ und n die bei den Verbindungen der Formel I angegebenen bevorzugten Bedeutungen haben.

Die Reaktion wird durchgeführt, indem man in eine Lösung des Oxims der Formel V in einem geeigneten Verdünnungsmittel portionsweise das Reduktionsmittel einträgt und durch gleichzeitige Zugabe einer Säure den pH-Wert des Reaktionsmediums im schwach sauren Bereich hält.

Die Reaktion wird bei -20 bis +100°C, bevorzugt bei 20°C, durchgeführt.

Es wird bei Normaldruck gearbeitet.

Die Ausgangskomponenten werden etwa in äquimolarem Verhältnis eingesetzt.

Es wird in Verdünnungsmitteln gearbeitet. Als solche seien genannt: Alkohole wie Methanol, Ethanol, Isopropanol, Ethylenglycol. Es kann auch in wäßrigen Alkoholen gearbeitet werden.

Es wird bei einem pH-Wert der Reaktionslösung von etwa 1-4 gearbeitet. Der pH-Wert kann durch eine geeichte Glaselektrode bestimmt werden. Ferner ist die Zugabe eines Farbindikators wie Bromkresolgrün oder Methylorange zur Einstellung des pH-Wertes geeignet.

Die Aufarbeitung kann in an sich bekannter Weise durch Ansäuern des Ansatzes mit konzentrierter Säure wie Salzsäure zur Zersetzung des Cyanoborhydrids und anschließendem Alkalisieren, gefolgt von einer Extraktion des N-Alkylhydroxylamins mit organischen Lösungsmitteln erfolgen (vgl. R. Borch J. Am. Chem. Soc. 93, 2897 (1971)).

Besonders vorteilhaft ist es jedoch, nach Beendigung der Borhydridreduktion und Zersetzung des Borhydrids mit konzentrierter Säure wie Salzsäure den Ansatz im Wasserstrahlvakuum einzuengen, den Rückstand mit Chloroform oder Dichlormethan oder Alkoholen wie Ethanol, Isopropanol oder Methanol zu extrahieren. Die organische Lösung des N-Alkylhydroxylamin-Hydrochlorids wird mit Natriumsulfat oder anderen geeigneten Trocknungsmitteln getrocknet und am Rotationsverdampfer eingeengt.

Die Verbindungen der Formel IV können auch in an sich bekannter Weise durch Reduktion der entsprechenden Nitroverbindungen mit Wasserstoff unter Katalyse von z.B. Palladium auf Kohle (vgl. US-P 3 173 953) oder durch Reduktion mit Zinkstaub Eisessig, mit amalgamiertem Aluminium, mit Zinn-(II)-chlorid (vgl. HOuben-Weyl, Methoden der org. Chemie Band 10/1 S. 1153) hergestellt werden.

Die Wirkstoffe werden als Leistungsförderer bei Tieren zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier-, und Hobbytieren verwendet.

Zu den Nutz-und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthähne, Tauben, Fische wie z.B. Karpfen, Forellen, Lachse, Aale, Schleien, Hechte, Reptilien wie z.B. Schlangen und Krokodile.

Zu den Zier-und Hobbytieren zählen Säugetiere wie Hunde und Katzen, Vögel wie Papageien, Kanarienvögel, Fische wie Zier-und Aquarienfische z.B. Goldfische.

Die Wirkstoffe werden unabhängig vom Geschlecht der Tiere während allen Wachstums-und Leistungsphasen der Tiere eingesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg, insbesondere 0,01 bis 5 mg/kg Körpergewicht pro Tag. Die passende Menge des Wirkstoffs sowie die passende Dauer der Verabriechung hängen insbesondere von der Art, dem Alter, dem Geschlecht, der Wachstumsund Leistungsphase, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums-und Leistungsphase temporär oder kontinuierlich verabreicht werden.

Bei kontinuierlicher Verabreichung kann die Anwendung ein-oder mehrmals täglich in regelmäßigen oder unregelmäßigen Abständen erfolgen.

Die Verabreichung erfolgt oral oder parenteral in dafür geeigneten Formulierungen oder in reiner Form.

Die Wirkstoffe können in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs-und Preßhilfsstoffen vorliegen.

Andere leistungsfördernde Wirkstoffe sind:

z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid.

Vitamine sind z.B. Vitamin A, Vitamin $D_3$, Vitamin E, B-Vitamine, Vitamin C.

Nicht-Protein-Verbindungen sind z.B. Biuret, Harnstoff.

Farbstoffe sind z.B. Carotinoide wie Citranaxanthin, Zeaxanthin, Capsanthin.

Antioxidantien sind z.B. Ethoxyquin, Butylhydroxy-Toluol.

Aromastoffe sind z.B. Vanillin.

Emulgatoren sind z.B. Ester der Milchsäure, Lecithin.

Fließhilfsstoffe sind z.B. Natriumstearat, Calciumstearat.

Konservierungsstoffe sind z.B. Zitronensäure, Propionsäure.

Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie-und Proteinversorgung sowie der Versorgung mit Vitaminen, Mineralsalzen und Spurenelementen sicherstellt.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4 \times H_2O$, 140 mg Zn $SO_4 \times 7\ H_2O$, 100 mg Fe $SO_4 \times 7\ H_2O$ und 20 mg Cu $SO_4 \times 5\ H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer-und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise bzw. Schweinen abgestimmt, sie können jedoch in gleicher oder ähnlicher Zusammensetzung auch zur Fütterung anderer Tiere verwendet werden.

Beispiel A

Ratten-Fütterungsversuch

Weibliche Laborraten 90-110 g schwer vom Typ SPF Wister (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammenetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt und die relative Gewichtszunahme im Vergleich zur unbehandelten Kontrolle errechnet.

Es werden die aus der Tabelle ersichtlichen Ergebnisse erhalten:

Tabelle 1

Ratten Fütterungsversuch

| Wirkstoff Beispiel Nr. | Wirkstoff Aufwand ppm | relative Gewichtszunahme % |
|---|---|---|
| 2 | 25 | 20 |
| 3 | 25 | 15 |
| 4 | 25 | 16 |
| 5 | 25 | 14 |
| 1 | 25 | 24 |
| 6 | 25 | 22 |
| 7 | 25 | 12 |
| 8 | 25 | 5 |
| 9 | 25 | 8 |
| 10 | 25 | 8 |

Beispiele zum Verfahren zur Herstellung der Verbindungen der Formel I

Beispiel 1

N-(2-(3,5-Dimethoxyphenyl)-2-hydroxyethyl)-N-2-(4-methoxyphenyl)-1-methylethyl)-hydroxylamin

In die Lösung von 1,1 g (3,1 mmol) C-(3,5-Dimethoxybenzoyl)-N-(2-(4-methoxyphenyl)-1-methylethyl)-nitron in 25 ml abs. Methanol wurden unter Kühlen portionsweise 250 mg (6, 2 mmol) $NaBH_4$ eingetragen. Man rührte 6 Stunden bei Raumtemperatur nach, säuerte unter Kühlung mit verdünnter Salzsäure an und dampfte ein. Der wäßrige Rückstand wurde mit Dichlormethan (3 × 10 ml) extrahiert, die vereinigten organischen Phasen wurden über $Na_2SO_4$ getrocknet und eingedampft. Man erhielt 900 mg ( 73 % d.Th.) des Hydrochlorids der Titelverbindung als amorphes, farbloses Pulver.

$^1$H-NMR (300 MHz, $CDCl_3$, $\delta$ (ppm)): 1,2 (d, 3H); 3,8 (s, 9H); 3,4-3,9 (m, 5H); (m, 1H); 6,4 (t,1H); 6,6 (dd, 2H); 6,8 (t, 2H); 7,1 (d, 2H).

Analog wurden die folgenden Verbindungen der Formel I hergestellt:

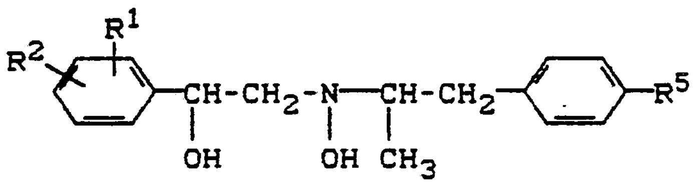

| Beispiel Nr. | $R^1$ | $R^2$ | $R^5$ | Schmelzp. [°C] |
|---|---|---|---|---|
| 2 | H | 3-Br | $OCH_2CH_2OH$ | amorph |
| 3 | H | 3-Br | $OCH_3$ | 55-58° C |
| 4 | H | 2-Cl | $OCH_3$ | amorph |
| 5 | $3\text{-}CH_3SO_2CH_2$ | 4-OH | $OCH_3$ | 60° C |
| 6 | $3\text{-}CH_3O$ | $4\text{-}CH_3O$ | $OCH_3$ | amorph |
| 7 | H | 3-Br | $OCH_2COOC_3H_7$ | amorph |
| 8 | H | 3-Br | $OCH_2COOCH_3$ | amorph |
| 9 | H | 3-Br | $COOCH_3$ | amorph |
| 10 | $3\text{-}CH_3O$ | $4\text{-}CH_3O$ | $COOCH_3$ | amorph |

Beispiele zum Verfahren zur Herstellung der Verbindungen der Formel II

Beispiel a1

C-(3-Brombenzoyl)-N-(2-(4-methoxyphenyl)-1-methylethyl)-nitron

2,31 g (10 mmol) 3-Bromphenylglyoxalhydrat, 2,175 g (10 mmol) N-(2-(4-Methoxyphenyl)-1-methylethyl)-hydroxylaminhydrochlorid und 820 mg (10 mmol) Natriumacetat wurden in 50 ml abs. Methanol 4 Stunden bei Raumtemperatur gerührt. Nach Filtration wurde eingedampft, der Rückstand mit 50 ml Wasser versetzt und mit Dichlormethan (3 × 25 ml) extrahiert.

Die vereinigten organischen Phasen wurden über $Na_2SO_4$ getrocknet und eingedampft. Der Rückstand kristallisierte beim Verreiben mit n-Pentan.

Ausbeute: 2,9 g (77 % d.Th.), Fp. 98°C

Analog wurden die folgenden Verbindungen der Formel II hergestellt:

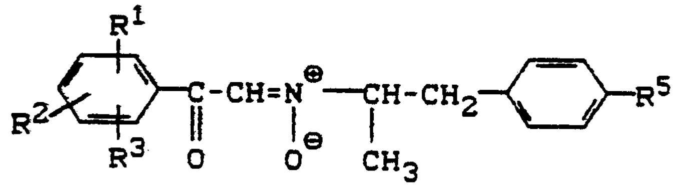

(II)

| Beispiel Nr. | $R^1$ | $R^2$ | $R^5$ | Fp. [°C] |
|---|---|---|---|---|
| $a_2$ | H | 2-Cl | $OCH_3$ | Öl |
| $a_3$ | $3-CH_3SO_2CH_2$ | 4-OH | $OCH_3$ | 67-68 |
| $a_4$ | $3-CH_3O$ | $5-CH_3O$ | $OCH_3$ | 92-95 |
| $a_5$ | $3-CH_3O$ | $4-CH_3O$ | $OCH_3$ | 134 |
| $a_6$ | H | 3-Br | $OCH_2COOCH_3$ | 108-110 |
| $a_7$ | H | 3-Br | $COOCH_3$ | 98-99 |
| $a_8$ | $3-CH_3O$ | $4-CH_3O$ | $COOCH_3$ | 148 |

Biespiele zum Verfahren zur Herstellung der Verbindungen der Formel IV

Beispiel b1

N-(2-(4-Methoxyphenyl)-1-methylethyl)hydroxylaminhydrochlorid

23,7 g (0,13 mol) 4-Methoxyphenylacetonoxim wurden in 200 ml Methanol gelöst und mit verdünnter Salzsäure auf pH 3 gestellt. Dann wurden portionsweise 8,3 g (0,13 mol) $NaBH_3CN$ eingetragen, wobei durch gleichzeitige Zugabe von verdünnter Salzsäure der pH bei 3 gehalten wurde. Man rührte über Nacht bei Raumtemperatur nach, stellte mit Salzsäure auf pH 1 und dampfte ein. Der wäßrige Rückstand wurde mit Dichlormethan (3 × 100 ml) extrahiert. Die vereinigten organischen Phasen wurden mit Wasser (3 × 25 ml) gewaschen, über $Na_2SO_4$ getrocknet und eingedampft.

Ausb. 23,3 g (80 % d.Th.), Fp. 124°C

Analog wurden die folgenden Verbindungen der Formel IV hergestellt:

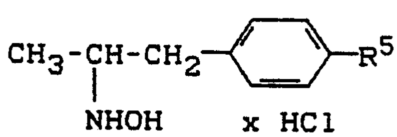

x HCl

(IV)

| Beispiel Nr. | $R^5$ | Fp. [°C] |
|---|---|---|
| $b_2$ | $OCH_2COOCH_3$ | Öl |
| $b_3$ | $COOCH_3$ | Öl |

**Ansprüche**

1. Arylethanolhydroxylamine der Formel I

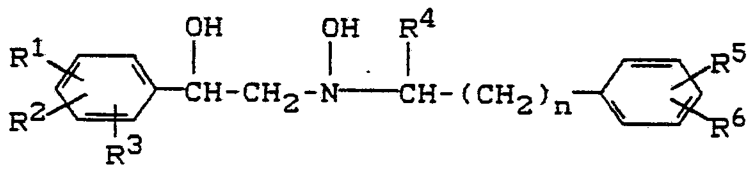

(I)

in welcher

$R^1$ für Halogen, CN, $NO_2$, Hydroxy, gegebenenfalls substituiertes Alkyl, Alkoxy oder Alkylthio ferner für Alkoxycarbonyl oder den Rest $-NR^7R^8$ steht,

$R^2$ für Wasserstoff, Halogen, CN, Hydroxy oder gegebenenfalls substituiertes Alkoxy steht, wobei die Reste $R^1$ und $R^2$ gemeinsam mit den angrenzenden C-Atomen einen gegebenenfalls substituierten Dioxolanyl- oder Dioxanylring bilden können,

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegebenenfalls substituiertes ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht,

sowie deren physiologisch verträgliche Salze, sterischen und optischen Isomeren.

2. Verfahren zur Herstellung der Arylethanolhydroxylamine der Formel I

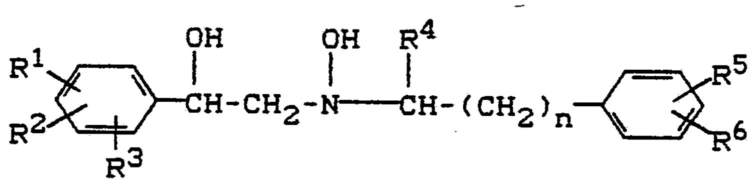

(I)

in welcher

$R^1$ für Halogen, CN, $NO_2$, Hydroxy, gegebenenfalls substituiertes Alkyl, Alkoxy oder Alkylthio ferner für Alkoxycarbonyl oder den Rest $-NR^7R^8$ steht,

$R^2$ für Wasserstoff, Halogen, CN, Hydroxy oder gegebenenfalls substituiertes Alkoxy steht,

wobei die Reste $R^1$ und $R^2$ gemeinsam mit den angrenzenden C-Atomen einen gegebenenfalls substituierten Dioxolanyl-oder Dioxanylring bilden können,

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht,

dadurch gekennzeichnet, daß man Arylglyoxalnitrone der Formel II

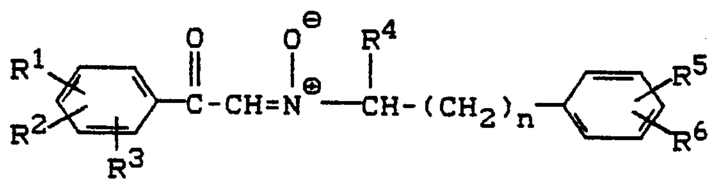
(II)

in welcher

$R^1$, $R^2$, $R^3$, $R^4$, $R^5$, $R^6$ und n die oben angegebene Bedeutung haben,

reduziert.

3. Arylglyoxalnitrone der Formel II

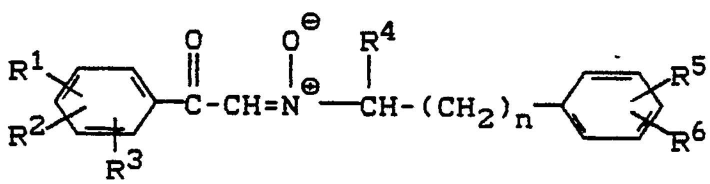
(II)

in welcher

$R^1$ für Halogen, CN, $NO_2$, Hydroxy, gegebenenfalls substituiertes Alkyl, Alkoxy oder Alkylthio ferner für Alkoxycarbonyl oder den Rest $-NR^7R^8$ steht,

$R^2$ für Wasserstoff, Halogen, CN, Hydroxy oder gegebenenfalls substituiertes Alkoxy steht, wobei die Reste $R^1$ und $R^2$ gemeinsam mit den angrenzenden C-Atomen einen gegebenenfalls substituierten Dioxolanyl- oder Dioxanylring bilden können,

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht.

4. Verfahren zur Herstellung der neuen Arylglyoxalnitrone der Formel II

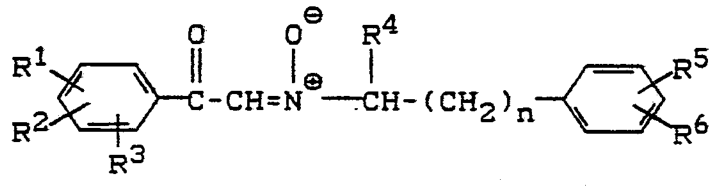

(II)

in welcher

$R^1$ für Halogen, CN, $NO_2$, Hydroxy, gegebenenfalls substituiertes Alkyl, Alkoxy oder Alkylthio, ferner für Alkoxycarbonyl oder den Rest $-NR^7R^8$ steht,

$R^2$ für Wasserstoff, Halogen, CN, Hydroxy oder gegebenenfalls substituiertes Alkoxy steht, wobei die Reste $R^1$ und $R^2$ gemeinsam mit den angrenzenden C-Atomen einen gegebenenfalls substituierten Dioxolanyl- oder Dioxanylring bilden können.

$R^3$ für Wasserstoff oder Halogen steht,

$R^4$ für Wasserstoff oder Alkyl steht,

$R^5$ für Wasserstoff, gegebenenfalls substituiertes Alkyl oder Alkoxy, Hydroxy, Halogen steht,

$R^6$ für Acyl, Acyloxy oder Alkoxy, das gegebenenfalls substituiert ist, steht,

$R^7$ für Wasserstoff oder Alkyl steht,

$R^8$ für Wasserstoff, Alkyl, Formyl (-CHO), Acyl, gegebenenfalls substituiertes Aryl oder Aralkyl steht,

n für 0, 1 oder 2 steht,

dadurch gekennzeichnet, daß man Arylgloxale der Formel III

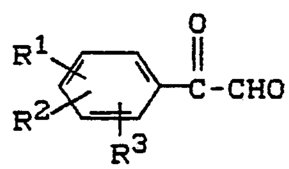

(III)

in welcher

$R^1$, $R^2$, $R^3$ die oben angegebene Bedeutung haben,

gegebenenfalls in Form ihrer Hydrate oder Addukte mit Alkoholen mit Hydroxylaminen der Formel IV

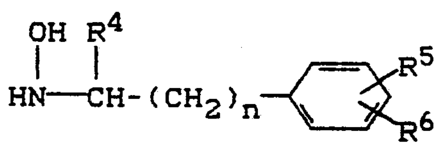

(IV)

in welcher

$R^4$, $R^5$, $R^6$, n die oben angegebene Bedeutung haben,

unter Wasserabsaltung umsetzt.

5. Hydroxylamine der Formel IV

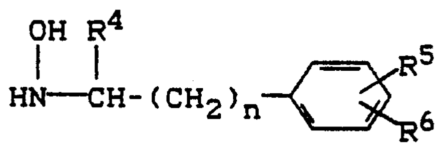

(IV)

in welcher

$R^4$, $R^5$, $R^6$, n die in Anspruch 3 angegebene Bedeutung haben.

6. Verfahren zur Herstellung der neuen Hydroxylamine der Formel IV

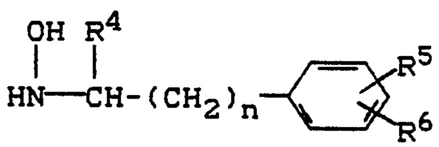

(IV)

dadurch gekennzeichnet, daß man Oxime der Formel V

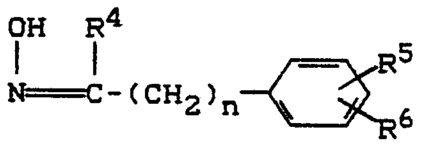

(V)

in welcher

$R^4$, $R^5$, $R^6$, n die oben angegebene Bedeutung haben,

reduziert.

7. Mittel zur Leistungsförderung von Tieren, gekennzeichnet durch einen Gehalt an Arylethanolhydroxylaminen der Formel I gemäß Anspruch 1.

8. Tierfutter, Trinkwasser für Tiere, Zusätze für Tierfutter und Trinkwasser für Tiere, gekennzeichnet durch einen Gehalt an Arylethanolhydroxylaminen der Formel I gemäß Anspruch 1.

9. Verfahren zur Herstellung von Mitteln zur Leistungsförderung von Tieren, dadurch gekennzeichnet, daß man Arylethanolhydroxylamine der Formel I gemäß Anspruch 1 mit Streck-und/oder Verdünnungsmitteln versetzt.

10. Verfahren zur Herstellung von Tierfutter, Trinkwasser für Tiere oder Zusätze für Tierfutter und Trinkwasser für Tiere, dadurch gekennzeichnet, daß man Arylethanolhydroxylamine der Formel I gemäß Anspruch 1 mit Futtermitteln oder Trinkwasser und gegebenenfalls weiteren Hilfsstoffen vermischt.